# EUROPEAN PATENT APPLICATION

(11) **EP 2 343 270 A2**
(43) Date of publication of application: **13.07.2011**
(21) Application number: 11163163.6
(22) Date of filing: 16.06.2006
(51) Int. Cl.: C07C 43/178, C07C 215/08, C07C 215/14, C07C 229/12, C07C 309/10, C07C 323/12, C07C 323/52, C07C 323/66, C08K 5/372, C08K 5/42, C09D 5/03, C09D 7/12, C11D 1/00, C23F 11/12, C10M 105/72, C10M 105/54, C10M 105/56, C10M 131/08, C10M 135/00, C10M 135/22

(54) **New fluorinated compounds, their synthesis and use**

(30) Priority: 20.06.2005 IT MI20051155
(62) Divisional of application: 06755959.1
(71) Applicant: GUARNIFLON S.p.A., 24060 Castelli Calepio (BG) (IT)
(72) Inventor: Maniero, Francesco, 30031, DOLO VE (IT); Padoan, Gennifer, 30015, CHIOGGIA VE (IT)
(74) Representative: Modiano, Micaela Nadia

(57) **Abstract**

New fluorinated compounds and method for their preparation. These compounds are used particularly but not exclusively as fluorinated surfactants or as solid lubricants to increase the characteristics of slipperiness and oil-hydro-repellence of surfaces that constitute hulls of watercraft and the like and/or their cladding materials, sleighs, chutes, bases of skis and the like. These compounds having the general formula (I) can be used as such or in solution, pure or in a mixture with other similar or different fluorinated compounds.

## Description

### Technical field

The present invention relates to new fluorinated compounds, to their synthesis and to their use particularly but not exclusively as surfactants and as slip agents.

### Background art

Fluorinated surfactants are currently widespread, since they reduce surface tension more than any other known surfactant. Currently, thanks to their particular properties, fluorinated surfactants are used for example in the field of extinguishing agents, such as extinguishing foams, because they increase the wettability of the extinguishing agent such as water. In particular, fluorinated surfactants have a film-forming action on the surface, blocking the combustible vapors and making it difficult for them to reignite. In formulations for extinguishing foams, fluorinated surfactants make the foam more fluid and therefore faster in spreading on the front of the fire and also more resistant to hydrocarbon pollution.

Categories of fluorinated surfactants, disclosed in scientific and patent publications, are already commercially available.

US4278552 and US4511733 disclose fluorinated amino acids obtained from the reaction between fluorinated epoxides and primary or secondary amines having the general formula HN(R2)-R1-COOQ. No reference is made to the possibility to replace the amines with other groups or to the possibility to react a fluorinated epoxide with an amine group in the absence of other acid groups or related salts on the organic molecule.

US6706923 discloses fluorinated iodo and/or alkenyl surfactants which use allyl glycidyl ether as epoxide group among the initial reagents for the synthesis. Besides relating to products that are different from the ones described here, US6706923 mentions neither the possibility to open the epoxide with hydroxyl groups nor the properties of the products that can be thus obtained.

US3172910 discloses fluorinated compounds having the formula Rf-(CH₂)ₘ-S-(CH₂)ₙCOOH. To produce these compounds (see, in this regard, FR2083422, US3686283, FR2113219, EP0526976A1, US4845300 and US6391948) it is first necessary to synthesize fluorinated thiols having the general formula Rf (CH₂) ₘSH, a phase which requires many onerous steps, including the addition of ethylene to the perfluoroalkyliodides CₙF₂ₙ₊₁I, the subsequent reaction with thiourea, and finally the corresponding basic hydrolysis of the addition product. These operations are generally accompanied by odorous emissions that cannot be neglected from the plant and environmental standpoint.

### Disclosure of the invention

The aim of the present invention is therefore to provide fluorinated compounds that overcome the drawbacks of known products.

An object is to provide fluorinated products having improved surfactant properties, a balance between oil- and water-repellence that makes them suitable for various uses, and a significant lubricating power.

Another object is to provide a method that is fast, inexpensive, safe, non-toxic and characterized by high yields, for the synthesis of the fluorinated products cited above.

Another object is to provide particularly advantageous uses of the fluorinated products cited above.

This aim and these and other objects are achieved by a fluorinated compound as claimed in claim 1.

This aim and these and other objects are also achieved by a method for synthesizing the fluorinated compounds according to the invention as claimed in claim 9.

This aim and these and other objects are also achieved by the use of the compounds according to the invention as claimed in claims 14 and 15.

### Ways of carrying out the invention

In a first aspect, the invention relates to new fluorinated compounds having the general formula (I) 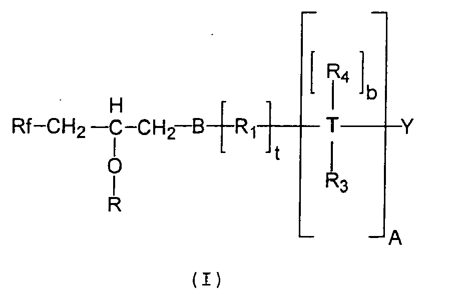 where
Rf is a saturated perfluorocarbon chain or a mixture of saturated perfluorocarbon chains which are preferably linear, optionally comprising ramified or cyclic portions, having the formula CₙF₂ₙ₊₁- in which n is an integer comprised between 4 and 20, preferably between 6 and 14, and more preferably is 6 or 8;
R is selected between hydrogen and a linear or ramified alkyl C₁-C₅ group, preferably R is CH₃- and more preferably R is H;
B is selected among:
   i) an ether group -O-,
   ii) a thioether group -S-,
   iii) an amine having the general formula 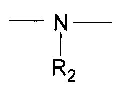 where R₂ is selected among:
      iii-a) hydrogen,
      iii-b) C₁-C₅ alkyl
      iii-c) a group having the formula 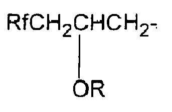 where R and Rf are as defined above,
R₁ is selected among:
   i) a linear or ramified group having the formula ― (C_{P}H₂p) ―, where p is an integer comprised between 1 and 50, preferably between 2 and 10;
   ii) a group having the formula ― (CₚH₂ₚ)_{q} ― C₆H₄ ― (Cₚ₁H₂ₚ₁) ᵣ ― where q and r are each independently 0 or 1, p1 is an integer comprised between 1 and 50 and p is as defined above;
   iii) a group having the formula - (CpH₂ₚ) _{q}-C₆H₄- (CH₂CH₂O) p- where p and q are as defined above and D is an integer comprised between 1 and 40;
   iv) a group having the formula 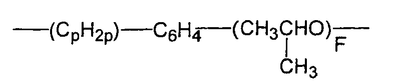 where p is defined above and F is an integer comprised between 1 and 40;
   v) a group selected among:
      v-a) 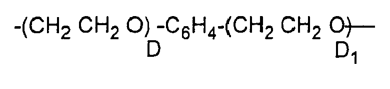
      v-b) 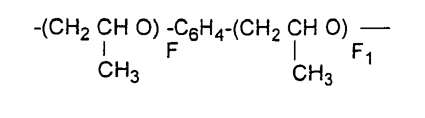
      v-c) 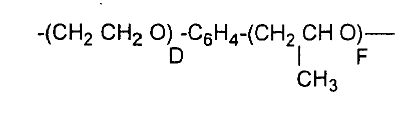 where D is as defined above, D₁ and F₁ are integers and each is independently comprised between 1 and 40;
   vi) a group having the formula -(CH₂CH₂O) _{D}- where D is defined above;
   vii) a group having the formula 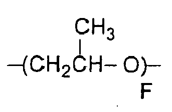 where F is as defined above;
   viii) an ethylene oxide and propylene oxide block copolymer having the formula 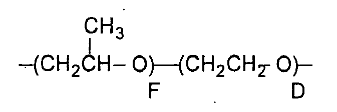 where F and D are as defined above;
   ix) an ethylene oxide-propylene oxide-ethylene oxide block copolymer having the formula 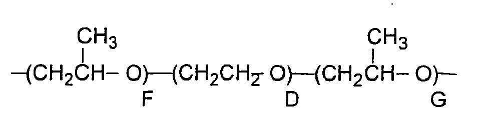 where F and D are as defined above, G is an integer comprised between 1 and 40;
t, A and b are each independently 0 or 1;
T is an atom of carbon or an atom of nitrogen;
R₃ is selected among:
   i) R, where R is as defined above,
   ii) a―PQ group where P is an anionic group or its corresponding neutral conjugate acid, and preferably is selected among:
      ii-a) -COOQ,
      ii-b) -SO₃Q,
      ii-c) -OSO₃Q,
         and Q is selected among:
      ii-d) hydrogen,
      ii-e) a cation of alkaline metals,
      ii-f) an ammonium ion, optionally mono-, di- or trisubstituted with one or more of:
         ii-f-1) C₁-C₁₀ alkyl, as CH₃-, (CH₃)₃C-, C₄H₉- C₈H₁₇-, preferably C1-C5 alkyl,
         ii-f-2) a group having the formula 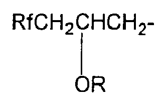 where R and Rf are as defined above, or
         ii-f-3) C₁-C₅ hydroxyalkyl;
   iii) a group -R₉-PQ where P and Q are as defined above and where R₉ is selected among:
      iii-a) a linear or ramified group having the formula - (Cₚ₉H₂₍ₚ₉₎)- where p9 is an integer comprised between 1 and 50, preferably between 2 and 10.;
      iii-b) a group having the formula - (Cₚ₉H₂₍ₚ₉₎) _{q9}-C₆H₄-(Cₚ₁₉H_{2 (p19)}) ᵣ₉‾ where q9 and r9 are each independently 0 or 1, p19 is an integer comprised between 1 and 50, and p9 is as defined above;
      iii-c) a group having the formula - (Cₚ₉H_{2(p9)q}9-C₆H₄-(CH₂CH₂O)D₉- where p9 and q9 are as defined above and D₉ is an integer comprised between 1 and 40;
      iii-d) a group having the formula 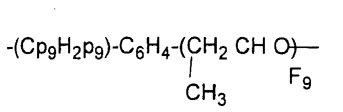 where p9 has been defined above and F₉ is an integer comprised between 1 and 40;
      iii-e) a group selected among:
         iii-e-1) 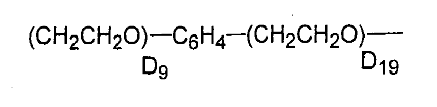
         iii-e-2) 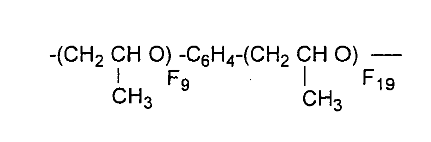
         iii-e-3) 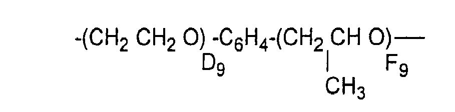 where D₉ and F₉ are as defined above, D₁₉ and F₁₉ are integers and each is independently comprised between 1 and 40;
      iii-f) a group having the formula - (CH₂CH₂0) _{D9}- where D₉ is as defined above;
      iii-g) a group having the formula 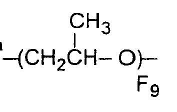 where F₉ is as defined above;
      iii-h) an ethylene oxide and propylene oxide block copolymer having the formula 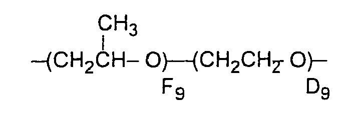 where F₉ and D₉ are as defined above;
      iii-i) an ethylene oxide-propylene oxide-ethylene oxide block copolymer having the formula 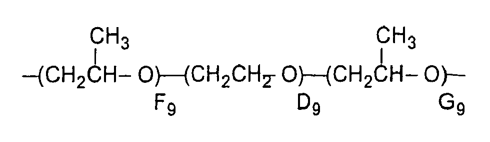 where F₉ and D₉ are as defined above and G₉ is an integer comprised between 1 and 40;
R₄ is hydrogen or a linear or ramified C₁-C₅ alkyl group; preferably methyl, more preferably H;
Y is hydrogen or a group having the formula (II) 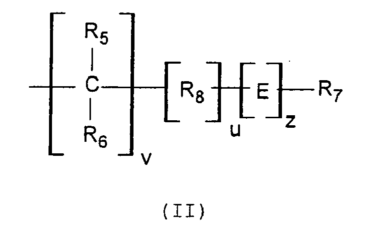 where
u, v and z are integers and each is independently equal to 0 or 1;
E is defined as B;
R₅ is hydrogen or a linear or ramified C₁-C₅ alkyl group; preferably methyl and more preferably H;
R₆ is selected among:
   i) R, where R is as defined above
   ii) a group -PQ, where P and Q are as defined above;
   iii) a group -R₁₀-PQ, where P and Q are as defined above and R₁₀ is selected among:
      iii-a) a linear or ramified group having the formula - (Cₚ₁₀H₂(_{P10})- where p₁₀ is an integer comprised between 1 and 50, preferably between 2 and 10;
      iii-b) a - (Cₚ₁₀H₂₍ₚ₁₀₎) _{q10}-C₆H₄- (Cₚ₁₁₀H₂ (ₚ₁₁₀) ) ᵣ10- group, where q10 and r10 are integers and each is independently equal to 0 or 1, p110 is an integer comprised between 1 and 50, p10 is
         as defined above;
      iii-c) a-(Cₚ₁₀H₂₍ₚ₁₀₎) _{q10}-C₆H₄-(CH₂CH₂O) _{D10}- group, where p10 and q10 are as defined above and D₁₀ is an integer comprised between 1 and 40;
      iii-d) a group having the formula 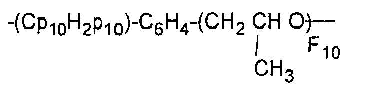 where p10 is as defined above and F10 is an integer comprised between 1 and 40;
      iii-e) a group selected among

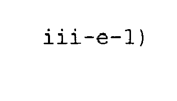

         iii-e-2)
         iii-e-3) 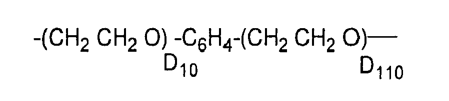 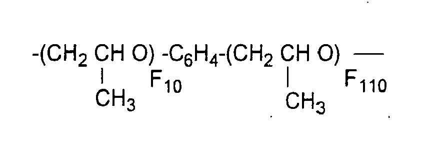 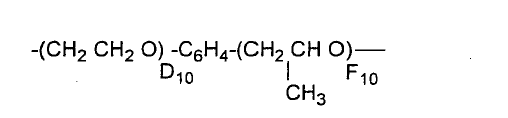 where D₁₀ and F₁₀ are as defined above, D₁₁₀ and F₁₁₀ are integers and each is independently comprised between 1 and 40;
      iii-f) a group having the formula -(CH₂CH₂O) _{D10}- where D₁₀ is as defined above;
      iii-g) a group having the formula 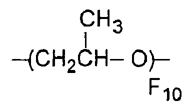 where F₁₀ is as defined above;
      iii-h) an ethylene oxide and propylene oxide block copolymer having the formula 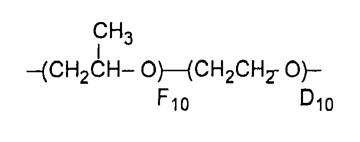 where F₁₀ and D₁₀ are as defined above;
      iii-i) an ethylene oxide-propylene oxide-ethylene oxide block copolymer having the formula 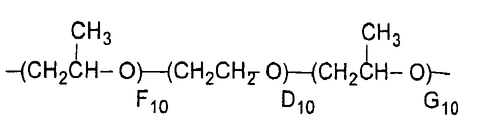 where F₁₀ and D₁₀ are as defined above and G₁₀ is an integer comprised between 1 and 40;
R₇ is selected between:
   i) hydrogen,
   ii) a group having the formula 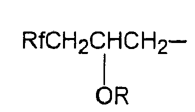 where Rf and R are as defined above;
R₈ is selected among:
   i) a linear or ramified group having the formula -(Cₚ₈H₂₍ₚ₈₎)-where p8 is an integer comprised between 1 and 50, preferably between 2 and 10;
   ii) a group having the formula - (Cₚ₈H₂₍ₚ₈₎)q8-C₆H₄-(Cₚ₁₈H₂₍ₚ₁₈₎)ᵣ₈- where q8 and r8 are each independently equal to 0 or 1, p18 is an integer comprised between 1 and 50, and p8 is as defined above;
   iii) a group having the formula -(Cₚ₈H₂₍ₚ₈₎) _{q8}-C₆H₄- (CH₂CH₂O) _{D8}- where p8 and q8 are as defined above and D₈ is an integer comprised between 1 and 40;
   iv) a group having the formula 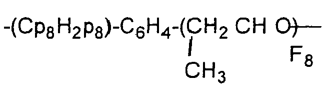 where p8 is as defined above and F₈ is an integer comprised between 1 and 40;
   v) a group selected among:
      v-a) 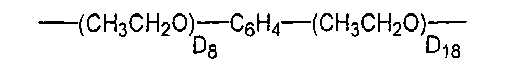
      v-b) 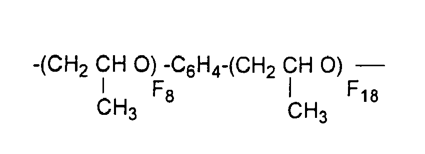
      v-c) 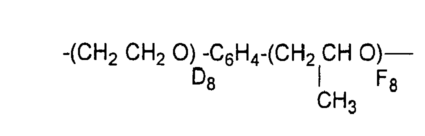 where D₈ and F₈ are as defined above, D₁₈ and F₁₈ are integers each independently comprised between 1 and 40;
   vi) a group having the formula -(CH₂CH₂O) _{D8}- where D₈ is as defined above;
   vii) a group having the formula 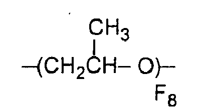 where F₈ is as defined above;
   viii) an ethylene oxide and propylene oxide block copolymer having the formula 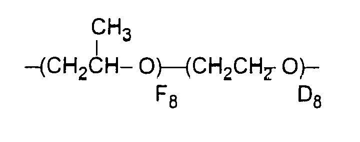 where F₈ and D₈ are as defined above;
   ix) an ethylene oxide-propylene oxide-ethylene oxide block copolymer having the formula 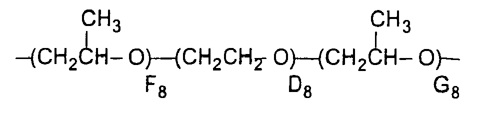 where F₈ and D₈ are as defined above and G₈ is an integer comprised between 1 and 40;
      wherein:
      - if T is carbon, then b is 1,
      - if T is nitrogen, then b is 0, and
      - if B is -N(R₂)-, then A and v are both equal to 0.

In a highly preferred embodiment, in which in the compounds having the formula (I) B is oxygen or sulfur, it has been found surprisingly that these compounds reduce to a greater extent than known products the surface tension of the formulations that contain them. Without intending to be bound to particular theories, it is believed that this may be due to the decrease of the polarity of the molecule and to its better orientation in the system.

In compounds having the formula (I) in which instead B is -NR₂-, and both A and v are equal to zero, they have proved to be surprisingly suitable as cationic surfactants for applications in water-based systems or as fluorinated solid lubricants.

Among the compounds having the formula (I), attention is called to the subgroup in which A is zero, and more preferably the subgroup in which t=0, v=0, z=0, u=1, R₈=-Cₚ₈H_{2P8}, R₇=CH₃-, a group having the general formula (III):

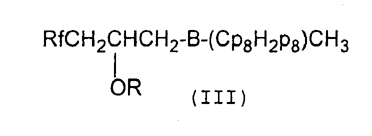

As also detailed hereinafter, the compounds having the formula (III) in which B is an ether or thioether bridge have physical properties which are similar to the traditional RF-RH waxes disclosed for example in US5202041, but their production process is faster and less expensive, so that their use is preferable with respect to said waxes. As also described hereinafter, the compounds having the general formula (I), and preferably the ones in which A and v are 0, have been found to be particularly suitable for use as slip agents to be used for example in sports activities on snow.

Compounds having the formula (I), especially but not exclusively in the cases in which there are two perfluorinated chains and with A=1, can be used advantageously in the papermaking sector to obtain oil- and water-repellent paper to be used in various fields but in particular in the food sector, the packaging sector, as labels, et cetera.

In the examples that follow, n, when present, can assume the values 4, 6, 8, 10, 12, 14, 16, 18. In one embodiment, the products from 1a) to 4b) in which n is present are constituted by a mixture of homologous products with a variable n, wherein n is selected among the discrete values cited above.

Preferred examples of compounds having the formula (I) are:
1a) 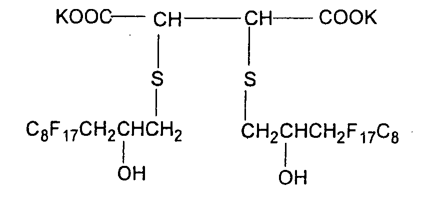
1b) 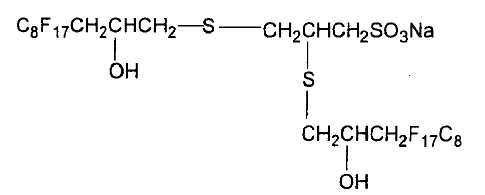
1c)

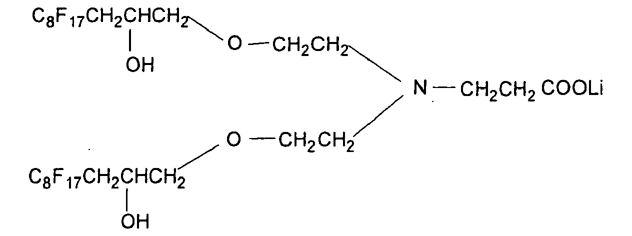

Preferred examples of compounds (I) with B and optionally E, equal to -S- are
2a) 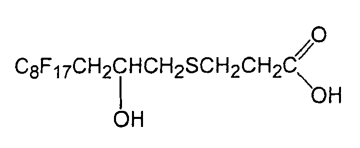
2b) 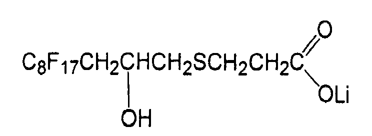
2c) 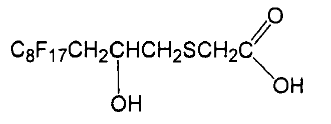
2d) 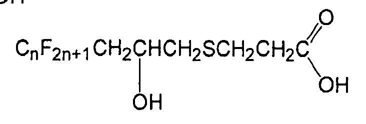
2e) 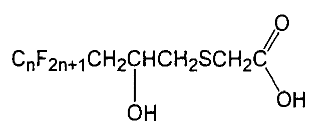
2f) 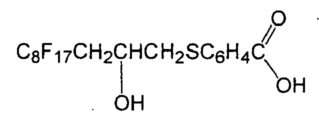
2g) 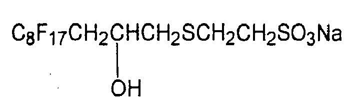
2h) 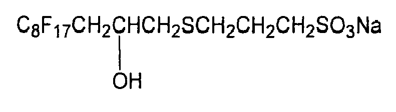
2i) 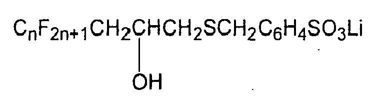
21) 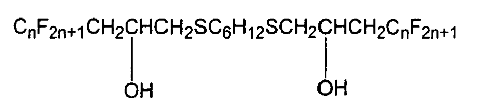
2m)

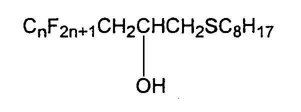

Specific examples of compounds (I) with B, and optionally E, equal to -O- are:
3a) 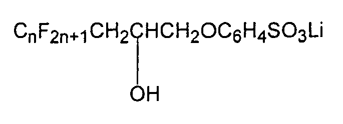
3b) 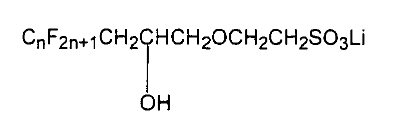
3c) 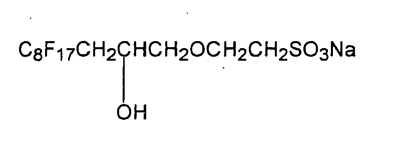
3d) 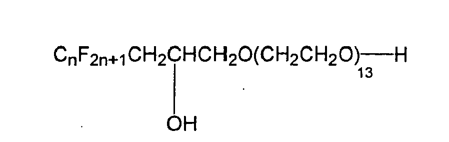 3e)

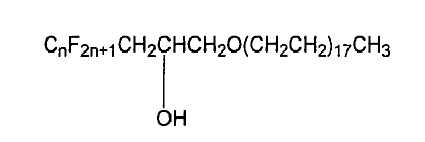

Specific examples of compounds (I) with B, and optionally E, equal to -N(R₂₎- are:
4a) 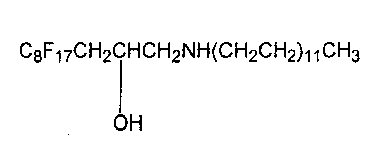
4b)

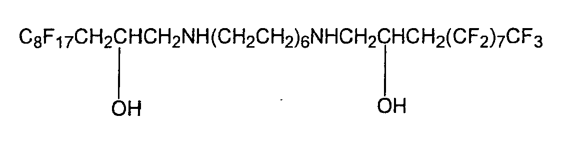

In another aspect, the invention relates to compositions comprising the compounds having the formula (I) as described above. The compounds having the general formula (I) can be used as such or in the form of compositions such as aqueous solutions or solutions of other solvents, such as alcohols such as methanol, ethanol, n-propanol, isopropanol, amyl alcohol, n-hexanol and the like, ketones such as acetone, methyl ethyl ketone, methyl isobutyl ketone, methyl-n-propyl ketone, diethyl ketone and the like, glycols such as diethylene glycol, propylene glycol and the like, esters such as ethyl acetate, butyl acetate, ethyl propionate, butyl propionate, dioxane, n-methyl pyrrolidinone, tetrahydrofuran, dimethylformamide, toluene, chloroform, methyl chloride, dichloromethane. These solvents can be used pure or in a mixture, in the presence of water or not.

Compounds having the general formula (I) can be used as such or with other fluorinated compounds which are similar or different, such as for example fluorinated and non-fluorinated amphoteric surfactants such as betaines, fluorinated and non-fluorinated anionic surfactants such as carboxylic or sulfonic acids, sulfuric esters, phosphates and phosphoric acids, thiosulfates et cetera, fluorinated and non-fluorinated cationic surfactants such as amine salts, quaternary amine compounds.

In the compositions that comprise the compounds of formula (I) in the field of paints and in preparations for extinguishing foams, the quantity of fluorinated compound (I) is comprised between 0.001% and 10% by weight, preferably between 0.001% and 5% and more preferably between 0.1% and 2% by weight on the weight of the final composition.

If the compounds (I) are used as solid lubricants for snow sports equipment, they can be used alone or in a mixture for example with paraffins, mixtures of paraffins, mixtures of paraffins and mineral oils, fluorinated compounds such as Rf-Rh and fluorinated compounds such as Rf-Rf, PTFE, boron nitride, molybdenum sulfide, fluorographite. In these mixtures the compound having the formula (I) is present in a quantity comprised between 0.3% and 30%, preferably between 3% and 15%.

Some particularly preferred compositions will be discussed hereinafter in the context of their specific applications.

Some of the characteristics of the invention might be defined in detail only with regard to a single product category comprised in the general formula (I). However, if not specified, the person skilled in the art will understand immediately, by relying on ordinary knowledge in the field, that the definition of these characteristics and advantages can be extended to all the compounds having the formula (I).

In another aspect, the invention relates to the use of the compounds having the formula (I) as defined above and of the compositions that comprise them. The compounds according to the invention, as such or in the form of compositions, have been found to be particularly suitable as agents designed to be applied to fluid and solid materials in order to give these materials a low surface tension.

The compounds having the formula (I) are therefore particularly suitable to be used:
- as non-specific fluorinated surfactants,
- as solid lubricants, particularly to increase the slipperiness and oil-hydro-repellence characteristics of surfaces that constitute hulls of watercraft and the like and/or their cladding materials, sleighs, chutes, ski bases, and the like;
- as additives in polymerization processes in emulsion,
- as corrosion inhibitors,
- as additives in coatings for wood, glass, metal, brick, cement, natural and synthetic stone,
- as additives in processes for treating paper, fabrics,
- as additives in enamels and in waxes for floors, for furniture and for shoes,
- as additives in cleaning products,
- as wetting agents,
- as additives in glass anti-mist formula,
- as additives for extinguishing agents and fire-resistant compositions.

In particular in the coating sector, it has been found that the fluorinated compounds having the formula (I), preferably the ones in which A is 1, more preferably the ones in which A is 1 and Y is hydrogen, surprisingly have a much higher performance than currently commercially available surfactants, since for an equal concentration with respect to known products they have numerous technical advantages. Among these advantages, mention can be made of:
- improvement of leveling and flow,
- improvement of gloss,
- reduction of the orange-peel phenomenon,
- increase in scratch-resistance,
- improvement of substrate wetting (better preventing the formation of craters and pinholes),
- better control of pigment separation
- improvement of corrosion resistance.

The compounds having the formula (I) where A is 1 can also be used with great advantages in the field of extinguishing agents and in particular in extinguishing foams.

A particularly interesting application of the compounds having the formula (I) is the preparation of fluoroprotein fire-retardant compositions. Emulsions based on hydrolyzed animal or plant protein are widely used in the fire-retardant foam sector, because they form very dense and stable foams. These products, however, have some disadvantages, since they form very compact foams, which are difficult to spread over the front of the fire and the resistance of the foam to the presence of hydrocarbons is limited. Accordingly, in order to improve the effectiveness of these protein emulsions, small quantities of fluorinated surfactants are incorporated, allowing to obtain a foam which is more fluid, less compact and more resistant to hydrocarbon contamination. The quantity of fluorinated surfactant to be introduced in the mixture is such as to have a final concentration generally comprised between 0.005% and 2%. Higher quantities are possible if required.

Moreover, fluorinated surfactants offer higher resistance to chemical attacks with respect to normal surfactants.

During specific tests conducted on the compounds according to the present invention, it has been found that in particular surfactants having the formula (I) and provided with a hydrophilic part, therefore in all cases in which A and/or v are different from zero, -B- and/or -E- are a -N(R₂₎- group, and/or in all cases in which there is one or more-(CH₂CH₂O)_{D}-, (CH₂CH(CH₃)O)_{F}- groups, allow to improve the characteristics of the formulations for extinguishing foams with respect to known surfactants. These characteristics are determined by means of some experimental tests, such as for example the measurement of the expansion ratio, i.e., the ratio between the generated foam volume and the volume of the initial aqueous solution, the settling time, i.e., the time required to recover one quarter of aqueous solution from the foam left to rest in a separation funnel, and the measurement of the fluidity of the foam, i.e., the rate at which the foam covers a preset portion of an inclined plane.

Compounds having the formula (I), preferably where A is 0, more preferably compounds having the formula (III), can be incorporated advantageously in the bases of skis in order to improve their inherent characteristics. Bases are constituted generally by high-density polyethylene and are manufactured by extrusion or sintering of the powdered polymer. Accordingly, bases can be formed by using a composition which comprises polyethylene powder and compounds having the formula (I), where these last are present in a percentage comprised between 2% and 10% by weight on the final composition. The resulting sheets have a lower surface tension value than polyethylene without additions.

Moreover, compounds having the formula (I) can be used as compositions in a mixture with paraffin waxes so as to improve their functional sliding characteristics. The mixture can be prepared by simple incorporation by melting, optionally with the addition of compatibility enhancing agents.

In another aspect, the invention relates to a method for the synthesis of fluorinated compounds having the formula (I) as defined above.

The compounds having the formula (I) to which the present invention relates can be synthesized easily starting from fluorinated epoxides having the general formula (AA): 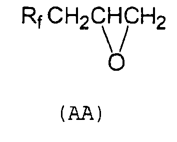 obtained in turn according to a per se known process, to which reference is made here (see for example Cirkva, Ameduri, Paleta in the Journal of Fluorine Chemistry 84 (1997) 53-57, Matuszczak, Feast in the Journal of Fluorine Chemistry 102 (2000) 269-277), which is simple and not very onerous.

The fluorinated epoxide obtained is then made to react with compounds having the general formula (BB): 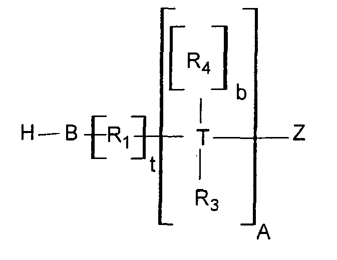 where Z is hydrogen or a group having the formula (CC): 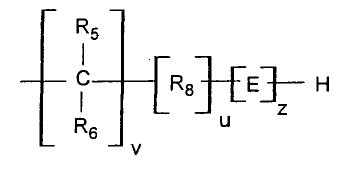 where the various variables are as defined above. Z differs from Y because for Y there is the variable R₇, which does not exist for Z. Therefore, the reaction between fluorinated epoxide and amine group described here is limited to the case in which no other acid polar groups and their salts are present in the organic molecule.

The addition of the two reagents occurs under different reaction conditions depending on the B and E groups that are involved.

In a first embodiment, B, and optionally E if present, are equal and are -S-. In this embodiment the reaction temperature must be comprised between 20°C and 120°C.

In this embodiment, the reaction can be performed in the mass or in the presence of an inert solvent such as water, ethanol, isopropanol, methyl ethyl ketone, tetrahydrofuran, dioxane, methyl isobutyl ketone, toluene, N methyl pyrrolidinone, alkanes, or mixtures thereof.

In a specific embodiment of the method in the case in which B and E are -S-, the reagents are placed in a reaction flask provided with mechanical agitation, thermometer, coolant in countercurrent, a flow of nitrogen and a thermostat-controlled oil bath. The reaction temperature is comprised between 20°C and 120°C, preferably between 40°C and 80°C, and the disappearance of the epoxide is monitored for example by gas chromatographic analysis. Usually, the reaction time varies between 4 and 20 hours, preferably between 6 and 12 hours.

If the compound having the formula (BB) contains acid groups of the -COOH, -S0₃H, or -OSO₃H type, it is possible to salify them preferably but non necessarily before the addition to the epoxide in order to improve their solubility by making them react with inorganic bases such as NaOH, KOH, LiOH, NH₄0H and mixtures thereof or with organic bases such as primary, secondary or tertiary amines and mixtures thereof. The use of inorganic bases or of tertiary amines can also precede the reaction of the reagent having the formula (BB) with the fluorinated epoxide (AA). The finished product can be placed in solution or can be kept as such.

In a different embodiment, B and optionally E if present are both -O- In this embodiment, it is necessary that Q, if present, be selected between a cation of alkaline metals and an ammonium ion trisubstituted by alkyl groups such as R₂.

It is also necessary for the reaction to occur in the presence of an inert solvent such as methyl ethyl ketone, tetrahydrofuran, dioxane, methyl isobutyl ketone, toluene, t-butyl methyl ether and mixtures thereof.

It is also necessary to provide, in the reaction environment, a Lewis acid, preferably in a quantity comprised between 0.001% and 1% by weight with respect to the mass of epoxide.

The reaction temperature must also be comprised between 20°C and 120°C.

In a specific embodiment of the method, in the case in which B and E are -O-, the compound having the formula (BB) is placed in a reaction flask provided with mechanical agitation, thermometer, coolant in countercurrent, stream of nitrogen and thermostat-controlled oil bath. In the reaction environment there must be a quantity of a Lewis acid, usually boron trifluoride etherate, in a quantity comprised between 0,001% and 1% by weight with respect to the mass of epoxide. The fluorinated epoxide or mixture of epoxides with n of different value is placed in a dripping funnel. The reaction temperature is comprised between 20°C and 120°C, preferably between 40°C and 80°C. At this temperature, the epoxide is added drop by drop to the reaction environment. The disappearance of the epoxide is monitored for example by gas chromatographic analysis. Usually, the reaction time varies between 4 and 20 hours, preferably between 6 and 12 hours. The product can be placed in solution or kept as such.

If B, and optionally E if present, are -N(R₂)- (the case in which A and v are equal to 0), the addition with the epoxide can occur in the mass or in the presence of inert solvents such as for example alcohols such as methanol, ethanol, isopropanol, glycols such as ethylene glycol, propylene glycol, ketones such as acetone, methyl ethyl ketone, methyl isobutyl ketone, esters such as butyl or ethyl acetate, and dioxane, tetrahydrofuran, N methyl pyrrolidinone and mixtures thereof.

The reaction temperature must be comprised between 20°C and 120°C; Q must also be selected among cations of alkaline metals and, if (BB) contains acid groups, they must first be salified with an equimolar quantity of a strong base.

In a specific embodiment of the method in the case in which B and E are -N(R₂)-, the reagents are placed in a reaction flask equipped with mechanical agitation, thermometer, coolant in countercurrent, flow of nitrogen and thermostat-controlled oil bath. The reaction temperature is comprised between 20°C and 120°C, preferably between 40°C and 80°C, and the disappearance of the epoxide is monitored for example by gas chromatographic analysis. Usually, the reaction time varies between 4 and 20 hours, preferably between 6 and 12 hours. The product can be placed in solution or kept as such.

If E is present, when B is different from E and one of the two is -O- while the other is a -S- group or a -N(R₂)- group, the different reactivity of the involved nucleophiles leads to a first addition of the fluorinated epoxide to the thioether or amine group and then, if required, also to the addition of the epoxide to the hydroxyl group. The conditions of addition to the individual functionalities are identical to the ones described above.

In the cases in which B and E are different from each other and neither is a -O- group, it is not possible to add selectively the epoxide first to one group with respect to the other. In this case it is necessary to add simultaneously the epoxide to both functionalities by following the methods already described.

In every embodiment, the method according to the invention leads to molecules having the formula (I) in which R is hydrogen. However, once the product of addition has been obtained, it is possible to functionalize the hydroxyl group with methods which are well-known in the field, so as to obtain all the other compounds having the formula (I).

The compounds having the formula (I) described above can also be used as monomers for synthesis of polymers of different kinds.

If one intends to obtain these polymers, R is not functionalized but remains hydrogen. In this way it is possible to use the -OH alcohol function as a reactive group for the subsequent synthesis of polymers such as for example polyurethanes and polyesters.

The polymers can be used to obtain an oil-hydro-repellence effect by treating various substrates such as paper, leather, fabrics and marble.

Other characteristics and advantages of the present invention will become better apparent from the description of the following preferred embodiments, intended merely as non-limiting examples. In all the examples that follow, the percentages are expressed as percentages by weight unless otherwise specified.

### Example 1

The product 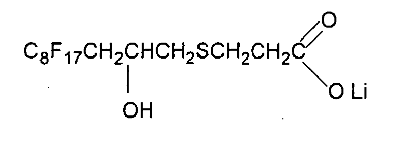 is prepared with the procedure described below.

The following are loaded into a four-neck 500 ml flask equipped with mechanical agitator, thermometer, countercurrent condenser, mounted on a temperature-controlled oil bath:
a) 15.00 g (0.14 mol) of 3-mercaptopropionic acid
b) 45 g of toluene
c) 18 g of water
d) 5.93 g (0.14 mol) of lithium hydroxide monohydrate.

After ten minutes of agitation at the temperature of 40°C, the reaction mixture is distilled to remove the solvent and the water present in the system. The following are added to the solid dehydrated product:
e) 66.64 g (0.14 mol) of fluorinated epoxide

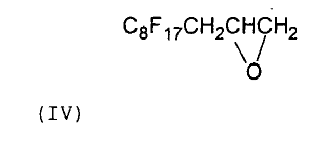

The temperature of the reaction mass is brought to 90°C. After one hour of reaction, a whitish paste has formed which receives the addition, after cooling to 70°C, of 82 g of 2-propanol. Reaction is performed in reflux for 3 more hours until the epoxide disappears completely. The reaction is monitored constantly with GC analysis, extracting the epoxide with a non-polar solvent. Finally, 164 g of water are added, obtaining a product with 25% active material with 25% 2-propanol and 50% water.

### Example 2

The following are loaded initially into a 1000 ml flask provided with the same services as in Example 1:
a) 50.00 g of 3-mercaptopropionic acid (0.47 mol)
b) 19.76 g of lithium hydroxide monohydrate (0.47 mol)
c) 69.7 g of water
d) 244.2 g of 2-propanol

Under agitation, and after complete solubilization, the solution is brought to 60°C and the following are introduced by means of a dripping funnel and in a nitrogen atmosphere:
e) 224.23 g of fluorinated epoxide (IV) (0.47 mol).

The reaction is monitored by gas chromatographic analysis, extracting the epoxide with a non-polar solvent such as chloroform. The reaction ends after 5 hours. The product is solubilized with 190.4 g of water and 309.8 g of 2-propanol. The solution thus obtained has 25% dry fraction, 25% water and 50% 2-propanol. It has a clear amber yellow color.

### Example 3

In the same manner as Example 2, but replacing the fluorinated epoxide (IV) with 245.34 g of a mixture of fluorinated epoxides having the general formula 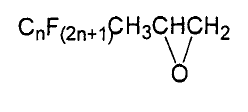 with the following composition:
58.0% with n=8
33.7% with n=10
8.3% with n=12
with an average molecular weight of 522 g/gmol.

At the end of the reaction, solubilization with water is performed so as to obtain a solution that comprises 25% dry fraction, 25% water and 50% 2-propanol. The solution is clear amber yellow.

### Example 4

The following product is synthesized:

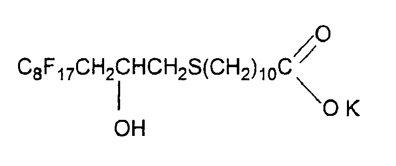

The following are loaded into the same apparatus as Example 1:
a) 21.84 g (0.1 mol) of 11-mercaptoundecanoic acid
b) 5.61 g (0.1 mol) of potassium hydroxide
c) 109.8 g of 2-propanol
d) 10 g of water

Under agitation and after complete solubilization, the pH of the solution is adjusted so as to obtain a value equal to 7-7.5.

The solution is brought to 60°C and the following is introduced by means of a dripping funnel and in a nitrogen atmosphere:
e) 47.6 g of epoxide (IV) (0.1 mol).

The reaction is monitored by gas chromatographic analysis, extracting the epoxide with a non-polar solvent such as chloroform. The reaction ends after 7.5 hours. The product is solubilized with 63.21 g of water and another 36.62 g of 2-propanol. The solution thus obtained has 25% dry fraction, 25% water and 50% 2-propanol. It has a clear yellow color.

### Example 5

The following product is synthesized:

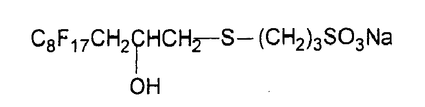

The following are introduced in the same apparatus used in Example 1 but in a 100 ml flask:
a) 13.3 g of epoxide (IV) (0.028 mol)
b) 5.0 g of the sodium salt of 3-mercaptopropansulfonic acid HS (CH₂) ₃SO₃Na (0.028 mol)
c) 5 g of water
d) 18.3 g of 2-propanol.

Under intense mechanical agitation and at the temperature of 75°C, complete disappearance of the epoxide occurs after 4.5 hours of reaction. The product is solubilized with 31.6 g of water so as to obtain a solution containing 25% dry fraction, 25% 2-propanol and 50% water.

### Example 6

The following product is synthesized: 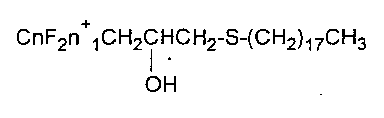 with the procedure described below.

The following components are loaded into the same apparatus of Example 1:
a) 47.60 g (0.1 mol) of epoxide having the formula (IV)
b) 28.66 g (0.1 mol) of 1-octadecanthiol;

These are made to react for 8.5 hours in an atmosphere of nitrogen at 145°C. The two compounds are immiscible when cold, but mix perfectly when hot, forming a single phase. After 8.5 hours, GC analyses show that the epoxide has disappeared completely.

One obtains a yellow product which is soluble in ketones such as acetone, methyl ethyl ketone, methyl isobutyl ketone and, when hot, in 2-propanol and in acetates such as butyl acetate and ethyl acetate.

### Example 7

The following product is synthesized:

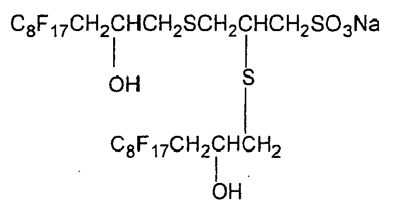

The same apparatus of Example 2 is loaded with:
a) 22.83 g (0.1 mol) of the sodium salt of 2,3-dimercapto-1-propansulfonic acid (Aldrich®);
b) 95.2 g (0.2moli) of fluorinated epoxide (IV)
c) 118 g of 2-propanol
d) 59 g of water

Under intense mechanical agitation and at the temperature of 75°C, the complete disappearance of the epoxide is observed after 5.5 hours of reaction. The product is solubilized with a further 59 g of water and 118 g of 2-propanol so as to obtain a solution containing 25% dry fraction, 50% 2-propanol and 25% water.

### Example 8

The following product is synthesized: 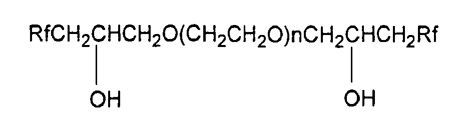 where Rf= C₈F₁₇-
19<n<20

The same apparatus of Example 2 provided with dripping funnel is loaded with:
a) 35 g (0.039 mol) of polyethylene glycol with an average molecular weight of 900 g/gmol (Aldrich®);
b) 45 g of dioxane.

The system is heated to 80°C, a temperature at which the reagent is perfectly soluble, and 200 µl of BF3 etherate are injected.

The following are added drop by drop
a) 37.0 g (0.078 mol) of epoxide (IV).

Dripping lasts for 1 hour. At the end of the addition, another 200µl of BF3 etherate are added. The reaction continues for another 2 hours at 80°C. The end product is homogeneous, pale yellow and soluble in water.

### Example 9

One proceeds as in Example 8, but the fluorinated epoxide (IV) is replaced with 40.71 g (0.078 mol) of a mixture of fluorinated epoxides having the general formula 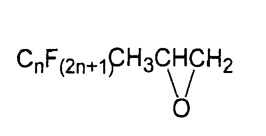 with the following composition:
58.0% with n=8
33.7% with n=10
8.3% with n=12
with an average molecular weight of 522 g/gmol.

The reaction in this case lasts 4 hours after the end of dripping.

### Example 10

The following product is synthesized:

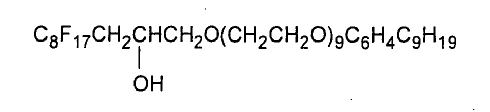

An apparatus as in Example 1 is loaded with:
a) 61.68 g (0.1 mol) of Tergitol® (Aldrich®);
b) 50 g of dioxane

Under agitation, the mixture is brought to 80°C and 200 µl of BF3 etherate are introduced. The following is loaded drop by drop in 30 minutes
c) 47.6 g (0.1 mol) of fluorinated epoxide (IV).

After dripping ends, one continues at the temperature of 80°C for 3 more hours, after which the epoxide has disappeared completely.

The dioxane is removed by distillation and with a slight flow of nitrogen and the product is solubilized with a 50:50 mixture of water and 2-propanol, obtaining a clear pale yellow solution with a 25% dry fraction.

### Example 11

The following product is synthesized: 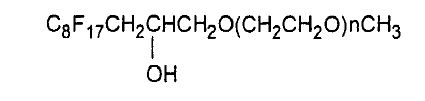 with 15<n<17

An apparatus as described in Example 1 is loaded with:
a) 75.0 g (0.1 mol) of polyethylene glycol methyl ether with an average molecular weight of 750 g/gmol (Aldrich®) ;
b) 50 g of dioxane.

Following the same procedure of Example 10, one obtains a clear white 25% solution of the product whose formula is cited above.

### Example 12

The following product is synthesized: 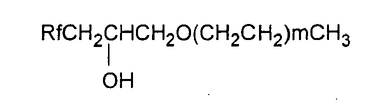 where Rf is CₙF₂₊₁- with the following composition:
58.0% with n=8
33.7% with n=10
8.3% with n=12
14<m<16

An apparatus as described in Example 1 is loaded with:
a) 90 g (0.196 mol) of polyethylene monoalcohol with mp=108°C, dr=0.985 and Mn=460 (Aldrich®)

The reaction mass is brought to 110-115°C so as to melt the product and 200 µl (102 g, 0.196 mol) of the mixture of fluorinated epoxides described in Example 3 are added.

The mixture of epoxides is loaded in one hour. The reaction mass is kept at this temperature and under agitation for 3 hours, during which two doses of BF3 etherate of 200 µl each are added.

The result is a straw yellow product, which melts in a temperature range between 85°C and 95°C, with a surface tension of 13.8 dynes/cm.

### Example 13

One proceeds as described in Example 12, using 93.3 g of fluorinated epoxide (IV). The result is a white product with a melting point comprised between 85°C and 90°C.

### Example 14

The following product is synthesized:

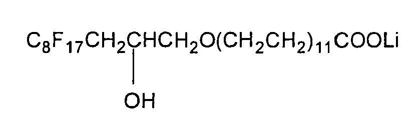

The same apparatus of Example 1 is loaded with:
a) 21.63 g (0.1 mol) of 12-hydroxydodecanoic acid;
b) 4.19 g of lithium hydroxide monohydrate
c) 50 g of methyl isobutyl ketone.

Under agitation, distillation is performed with a Marcusson-type apparatus at the azeotropic temperature of 92°C to remove the water that is present in the system. Drop by drop, at the temperature of 85°C, 200 ul of BF3 etherate are introduced together with
b) 47.6 g (0.1 mol) of fluorinated epoxide (IV).

Dripping lasts one hour, continuing subsequently with the reaction for another 4.5 hours at 85°C, adding two 200 µl doses of BF3 etherate.

200 g of water are added and then the solvent is distilled with part of the water added at 92°C with a flow of nitrogen. Finally, 231 g of 2-propanol are loaded and the quantity of water removed during distillation is restored. The result is a pale yellow homogeneous product with 15%-16% dry material.

### Example 15

The following product is synthesized:

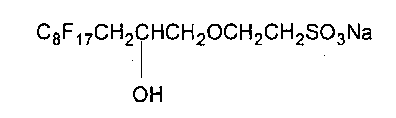

The same apparatus of Example 1 is loaded with:
a) 14.81 g (0.1 mol) of sodium salt of isoethionic acid (Aldrich®) having the formula HOCH₂CH₂SO₃Na;
b) 12 g of dioxane.

Under intense agitation, this mixture is brought to 90°C. At this temperature, by means of a dripping funnel, the following is dripped:
c) 47.6 g (0.1 mol) of fluorinated epoxide (IV).

Before dripping, 200 µl of BF3 etherate are added to the reaction mass. When dripping ends, another 400 µl of BF3 etherate is added in two separate doses. The reaction temperature is 90°C. The disappearance of the fluorinated epoxide is monitored by GC analysis. The reaction is finished after 6.5 hours.

### Example 16

The following product is synthesized: 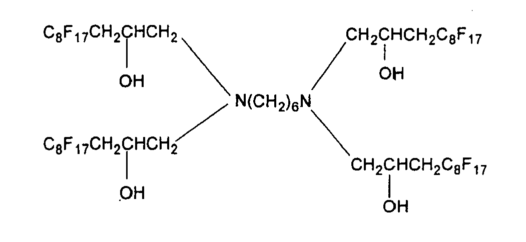 An apparatus similar to the one of Example 1 is loaded with:
a) 2.8 g (0.024 mol) of hexamethylenediamine
b) 45.70 g (0.096 mol) of epoxide having the formula (IV).

The reaction is performed in the mass at the temperature of 120°C for 7 hours. The product, when cold, is an orange solid with a melting point comprised between 60°C and 65°C and a surface tension of 13.4 mN/m. The product is dissolved in 2-propanol until a 50% solution is obtained.

### Example 17

The following product is synthesized:

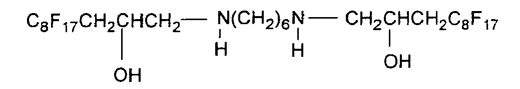

Using the same procedure as in the preceding example, the following are made to react:
a) 2.8 g (0.024 mol) of hexamethylenediamine
b) 22.85 g (0.048 mol) of epoxide having the formula (IV)

The reaction is performed at 100°C for 3 hours. GC analysis shows that the intended product has 81% concentration; 9.5% is the product [C₈F₁₇CH₂CH(OH)CH₂]₂N(CH₂)₆NHCH₂CH(OH)CH₂C₈F₁₇ and the remaining percentage is the monosubstituted product C₈F₁₇CH₂CH(OH)CH₂NH(CH₂)₆NH₂. The mixture of these products is solid, with a melting point comprised between 45°C and 55°C. The product can be purified by dissolving it when hot in tetrahydrofuran. When cold, the intended product precipitates and is filtered. The yield is 66%. The surface tension of the product is 7.6 dynes/cm.

### Example 18

The following product is synthesized:

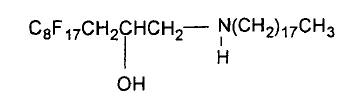

The same apparatus of Example 1 is loaded with:
a) 26.95 g (0.1 mol) of octadecylamine
b) 47.60 g (0.1 mol) of epoxide having the formula (IV).

The reaction is performed at 120°C for 3.5 hours. GC analysis shows that the intended product has a concentration of approximately 90%, while the remaining percentage is a mixture of unreacted amine and disubstituted amine. The end product has a melting point of 68°C-70°C. It can be placed in an aqueous solution by adding 6.0 g of acetic acid, 161.0 g of 2-propanol and 80.5 g of water. It is a good cationic surfactant, which at a concentration of 0.01% by weight in water provides a surface tension of 19.5 mN/m.

### Example 19

The surface tensions of various molecules are measured. Table 1 lists the surface tension measurements for some of the products having the formula (I) that have become evident in the field of paints and in the field of solid lubricants for snow gear.

**Table 1**

| | Surface tension or aq solutions [dynes/cm] | | |
|---|---|---|---|
| Product in solution | Concentration in deionized water at 25°C | | |
| | 0.001% | 0.01% | 0.1% |
| Comparison Example 1 | | | |
| F(CF₂CF₂)x(CH₂)₂S (CH₂)₂COOLi x = 1 to 9 | 48 | 22 | 18 |
| Product of Example 1 | 44 | 21 | 16 |
| Comparison Example 2 | | | |
| RfCH₂CH₂O(CH₂CH₂O)ₓH | 29 | 24 | / |
| where: | | | |
| Rf is F(CFCF)y- | | | |
| x is from 0 to approx. 25 | | | |
| y is from 1 to approx. 9 | | | |
| Product of Example 11 | 27 | 19 | / |
| Comparison Example 3 | | | |
| C₆F₁₃CH₂CH₂SO₃NH₄ | 69 | 44 | 24 |
| Product of Example 5 | 45 | 25 | 19 |
| | | | |

| Solid products | Surface tension of the solid [dynes/cm] | | |
|---|---|---|---|
| Comparison Example 4 | | | |
| C₈F₁₇C₁₈H₃₇ | 16.6 | | |
| Product of Example 12 | 13.8 | | |
| Comparison Example 5 | | | |
| C₈F₁₇C₆H₁₂C₈F₁₇ | 9.2 | | |
| Product of Example 17 | 7.6 | | |

Table 1 relates to products having the formula (I) that have structural analogies with respect to commercially available ones (comparison Examples 1 to 5) so as to best highlight the superior properties of the former.

Further tests carried out in testing laboratories have subsequently confirmed that even in cases in which the difference in surface tension between the surfactants according to the invention and known ones was slight, in practice the surfactants having the formula (I) entailed distinctly better results. For example, a conspicuous reduction of the orange peel effect and of pinholes, together with a higher scratch resistance, have been observed. One of these "visual" tests related to the semiempirical assessment of the influence of the fluorinated surfactant on the flow of paints. The results are given in Table 2:

**Table 2**

| | Clear coating for building | Pigmented coating for building | Glossy coating for wood | Satin coating for wood |
|---|---|---|---|---|
| Assessed product | Assessment | | | |
| Comparison Example 1 | | | | |
| F(CF₂CF₂)x(CH₂) ₂S (CH₂) ₂COOLi x = 1 to 9 | 1 | 1 | 4 | 2 |
| Product of Example 1 | 5 | 5 | 5 | 3 |
| Comparison Example 2 | | | | |
| RfCH₂CH₂O (CH₂CH₂O)xH | 2 | 2 | / | / |
| where: | | | | |
| Rf is F(CF₂CF₂)y- | | | | |
| x is from 0 to approx. 25 | | | | |
| y is from 1 to approx. 9 | | | | |
| Product of Example 11 | 4++ | 4 | / | / |

where the assessment ranges from O = very bad to 5 = excellent. The various surfactants were tested at equal concentrations.

The fact that the assessments given above regarding the effectiveness of the surfactants were performed with samples of coating which were very different from each other shows even more clearly that the fluorinated products according to the invention allow to achieve valid results over a wide range of quantity used and even in very different systems and fields of application.

### Example 20

Assessment of the influence of the presence of the fluorinated surfactants on fire suppression foams.

A protein concentrate was prepared which was intended for use in extinguishing foams and had the following formulation:

| | |
|---|---|
| Protein concentrate at 53% by weight | 100 g |
| water | 27 g |
| 2-propanol | 7 g |
| sodium dodecylbenzene sulfonate at 30% by weight | 9.5 g |
| sorbitan monolaurate (HLB 8.6) | 4 g |
| fluorinated surfactant at 25% by weight | 5 g |

6 g of this concentrate is taken and diluted with 94 g of deionized water. The characteristics of the foam were determined according to a number of simple experiments (cited hereafter), which allow to check the influence of the addition of the fluorinated surfactant on the foam.

### a) Expansion ratio (TE)

100 ml of diluted solution are converted into foam by means of a mechanical agitator with two whips. The expansion ratio TE is the ratio TE=V/v, where V is the final foam volume and v is the volume of the initial solution converted completely into foam;

### b) Settling time (TD)

This is defined as the time required to collect in a graduated cylinder a volume of liquid that corresponds to one quarter of the volume of solution converted into foam, i.e., in the case, 25 ml;

### c) Foaming power (PS)

This is defined as the volume of the foam formed by pouring 500 ml of foaming solution from a dripping funnel with an output diameter of 13 mm arranged at a height of 450 mm on 100 ml of said solution contained in a 2-liter container. The volume of the foam is measured 30 seconds after the introduction of the 500 ml of solution;

### d) Foam fluidity (FS)

The fluidity of the foam is determined by measuring the speed with which the foam flows on a plane that is inclined by 10°. This inclined plane is the bottom of a foam containment tray, which is 35 cm long, 21 cm wide and 10 cm high. This portion of the tray is separated from a second container by a movable panel. This last container contains 2 liter of foam. The time taken by the foam to travel along the entire inclined plane of the tray is measured from the moment when the movable panel is lifted.

The fluorinated surfactant marketed under the name FORAFAC®1157 was considered as a reference for the tests:

| | Concentrate with FORAFAC®1157 | Concentrate with product of Example 2 | Concentrate with product of Example 5 | Concentrate with product of Example 10 | Concentrate with product of Example 11 |
|---|---|---|---|---|---|
| TE | 8.5 | 9 | 9 | 8.5 | 9 |
| TD(sec) | 300 | 360 | 370 | 330 | 340 |
| PS(ml) | 300 | 320 | 380 | 400 | 400 |
| FS(sec) | 14 | 10 | 12 | 9 | 10 |

### Example 22

The assessment of the effectiveness of the compounds of Examples 6 and 12 was made on a Nordic skiing trail traced with a constant slope and whose path was at an altitude of 1400 m. Weather and snow conditions were as follows:
- air temperature: -1
- relative humidity %: 60
- snow temperature: -4
- snow type: transformed
- snow appearance: grainy
- weather conditions: overcast

A photocell was placed at the beginning and at the end of the trail to measure travel times. The tests were conducted by a ski instructor, expert in performing this kind of test. Four pairs of skis suitable for Nordic skiing races were used; all were treated with MAPLUS P1 MED® paraffin-based ski wax, and four products, specified below as A, B, C, D, were applied to them by following the standard application procedure of these products, which is known in the field. The four applied products are composed as follows:
- product A: semiperfluorinated alkanes having the general formula RF-RH, with C₂₈-C₃₂ linear chains, prepared according to Example 2 of US5202041;
- product B: asymmetric aromatic diester of terephthalic acid, in particular (asymmetric) octadecyl heptadecafluoroundecyl terephthalate
- product C: product of Example 12
- product D: product of Example 6

The skier performed five series of tests (five laps of the trail) for each product, after resetting the skis according to the standard procedure used by skimen.

The results have allowed to point out that the average of the lap times when using skis treated with C is 0,24% lower than with product A, 0,29% lower than with product B, and 0.15% lower than with product D.

Although the text has described only some preferred embodiments according to the invention, the person skilled in the art will realize that numerous variations of the embodiments described in the text and in the examples that follow are possible without thereby altering the inventive concept of the present invention.

The disclosures in Italian Patent Application No. MI2005A001155, from which this application claims priority, are incorporated herein by reference.

## Claims

1. A fluorinated compound having the general formula (I) 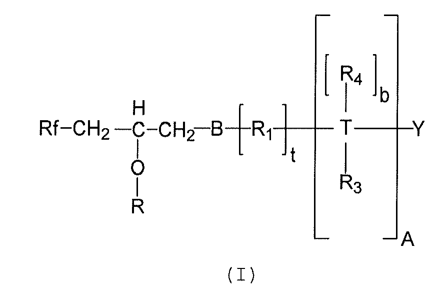 where
Rf is a saturated perfluorocarbon chain or a mixture of saturated perfluorocarbon chains which are preferably linear, optionally comprising ramified or cyclic portions, having the formula CₙF₂ₙ₊₁- in which n is an integer comprised between 4 and 20, preferably between 6 and 14, and more preferably is 6 or 8;
R is selected between hydrogen and a linear or ramified alkyl C₁-C₅ group, preferably R is CH₃- and more preferably R is H;
B is
a thioether group -S-,
R₁ is selected among:
i) a linear or ramified group having the formula -(CₚH₂p)-, where p is an integer comprised between 1 and 50, preferably between 2 and 10;
ii) a group having the formula - ( CpH₂ₚ)_{q}-C₆H₄- (Cₚ₁H₂ₚ₁) ᵣ- where q and r are each independently 0 or 1, p1 is an integer comprised between 1 and 50 and p is as defined above;
iii) a group having the formula -(C_{P}H₂ₚ)q-C₆H₄-(CH₂CH₂O)_{D}- where p and q are as defined above and D is an integer comprised between 1 and 40;
iv) a group having the formula 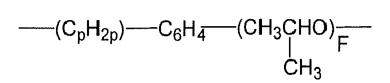 where p is defined above and F is an integer comprised between 1 and 40;
v) a group selected among:
v-a) 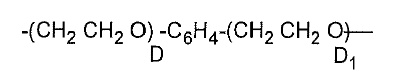
v-b) 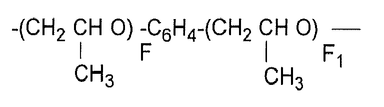
v-c) 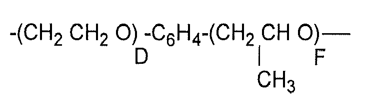 where D is as defined above, D₁ and F₁ are integers and each is independently comprised between 1 and 40;
vi) a group having the formula -(CH₂CH₂O)_{D}- where D is defined above;
vii) a group having the formula 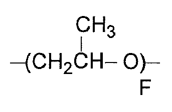 where F is as defined above;
viii) an ethylene oxide and propylene oxide block copolymer having the formula 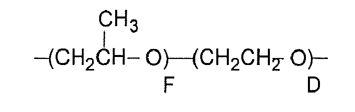 where F and D are as defined above;
ix) an ethylene oxide-propylene oxide-ethylene oxide block copolymer having the formula 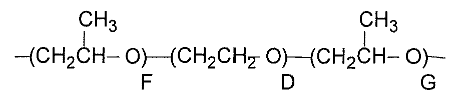
where F and D are as defined above, G is an integer comprised between 1 and 40;
t, A and b are each independently 0 or 1;
T is an atom of carbon or an atom of nitrogen;
R₃ is selected among:
i) R, where R is as defined above,
ii) a group -PQ where P is an anionic group or its corresponding neutral conjugate acid, and preferably is selected among:
ii-a) -COOQ,
ii-b) -SO₃Q,
ii-c) -OSO₃Q,
and Q is selected among:
ii-d) hydrogen,
ii-e) a cation of alkaline metals,
ii-f) an ammonium ion, optionally mono-, di- or trisubstituted with one or more of:
ii-f-1) C₁-C₁₀ alkyl, as CH₃-, (CH₃) ₃C-, C₄H₉-, C₈H₁₇-, preferably C1-C5 alkyl,
ii-f-2) a group having the formula 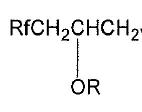 where R and Rf are as defined above, or
ii-f-3) C₁-C₅ hydroxyalkyl;
iii) a group -R₉-PQ where P and Q are as defined above and where R₉ is selected among:
iii-a) a linear or ramified group having the formula - (cₚ₉H₂₍ₚ₉₎)- where p9 is an integer comprised between 1 and 50, preferably between 2 and 10;
iii-b) a group having the formula - (Cp₉H_{2 (p9)}) q₉-C₆H₄-(Cₚ₁₉H₂₍ₚ₁₉₎) ᵣ₉- where q9 and r9 are each independently 0 or 1, p19 is an integer comprised between 1 and 50 and p9 is as defined above;
iii-c) a group having the formula - (Cp₉H₂₍ₚ₉₎) _{q9}-C₆H₄-(CH₂CH₂O)_{D9}- where p9 and q9 are as defined above and D₉ is an integer comprised between 1 and 40;
iii-d) a group having the formula 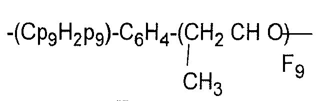 where p9 has been defined above and F₉ is an integer comprised between 1 and 40;
iii-e) a group selected among:
iii-e-1) 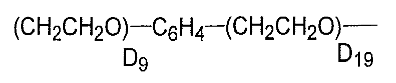 iii-e-2) 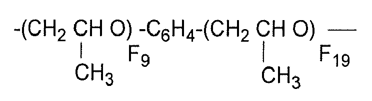
iii-e-3) 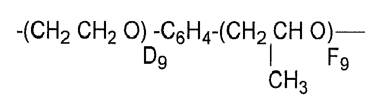 where D₉ and F₉ are as defined above, D₁₉ and F₁₉ are integers and each is independently comprised between 1 and 40;
iii-f) a group having the formula - (CH₂CH₂O) _{D}9- where D₉ is as defined above;
iii-g) a group having the formula 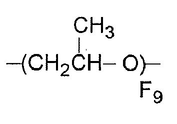 where F₉ is as defined above;
iii-h) an ethylene oxide-propylene oxide block copolymer having the formula 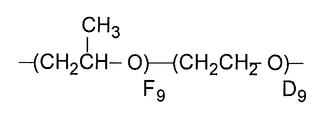 where F₉ and D₉ are as defined above;
iii-i) an ethylene oxide-propylene oxide-ethylene oxide block copolymer having the formula 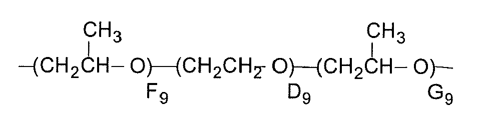 where F₉ and D₉ are as defined above and G₉ is an integer comprised between 1 and 40;
R₄ is hydrogen or a linear or ramified C₁-C₅ alkyl group; preferably methyl, more preferably H;
Y is hydrogen or a group having the formula (II) 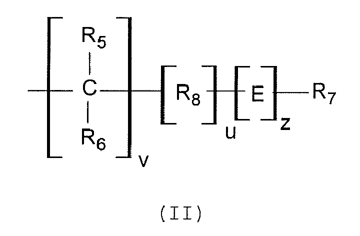 where
u, v and z are integers and each is independently equal to 0 or 1;
E is defined as B;
R₅ is hydrogen or a linear or ramified C₁-C₅ alkyl group; preferably methyl and more preferably H;
R₆ is selected among:
i) R, where R is as defined above
ii) a group -PQ, where P and Q are as defined above;
iii) a group -R₁₀-PQ, where P and Q are as defined above and R₁₀ is selected among:
iii-a) a linear or ramified group having the formula - (Cₚ₁₀H₂₍ₚ₁₀₎)- where p10 is an integer comprised between 1 and 50, preferably between 2 and 10;
iii-b) a - (Cₚ₁₀H_{2 (p10)}) _{q10}-C₆H₄- (Cₚ₁₁₀H₂₍ₚ₁₁₀₎) ᵣ₁₀- group, where q10 and r10 are integers and each is independently equal to 0 or 1, p110 is an integer comprised between 1 and 50, p10 is as defined above;
iii-c) a - (Cₚ₁₀H2 ₍ₚ₁₀₎) _{q10}-C₆H₄- (CH₂CH₂O) _{D10}- group, where p10 and q10 are as defined above and D₁₀ is an integer comprised between 1 and 40;
iii-d) a group having the formula 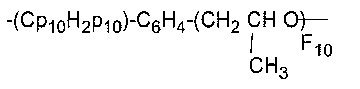 where p10 is as defined above and F10 is an integer comprised between 1 and 40;
iii-e) a group selected among
iii-e-1) 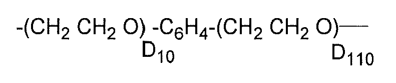 iii-e-2) 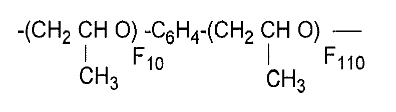
iii-e-3) 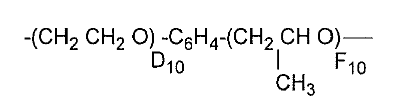 where D₁₀ and F₁₀ are as defined above, D₁₁₀ and F₁₁₀ are integers and each is independently comprised between 1 and 40;
iii-f) a group having the formula -(CH₂CH₂O) _{D10}- where D₁₀ is as defined above;
iii-g) a group having the formula 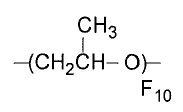 where F₁₀ is as defined above;
iii-h) an ethylene oxide and propylene oxide block copolymer having the formula 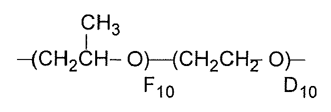 where F₁₀ and D₁₀ are as defined above;
iii-i) an ethylene oxide-propylene oxide-ethylene oxide block copolymer having the formula 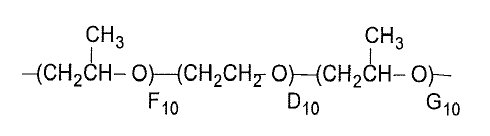 where F₁₀ and D₁₀ are as defined above and G₁₀ is an integer comprised between 1 and 40;
R₇ is selected between:
i) hydrogen,
ii) a group having the formula 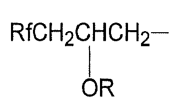 where Rf and R are as defined above;
R₈ is selected among:
i) a linear or ramified group having the formula -(Cₚ₈H₂₍ₚ₈₎)-where p8 is an integer comprised between 1 and 50, preferably between 2 and 10;
ii) a group having the formula - (Cₚ₈H_{2 (p8)}) _{q8}-C₆H₄- (Cₚ₁₈H₂₍ₚ₁₈₎₎ r8- where q8 and r8 are each independently equal to 0 or 1, ₚ18 is an integer comprised between 1 and 50, and p8 is as defined above;
iii) a group having the formula - (Cₚ₈H₂₍ₚ₈₎) _{q8}-C₆H₄- (CH₂CH₂O) _{D8}- where p8 and q8 are as defined above and D₈ is an integer comprised between 1 and 40;
iv) a group having the formula 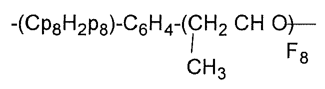 where p8 is as defined above and F₈ is an integer comprised between 1 and 40;
v) a group selected among:
v-a) 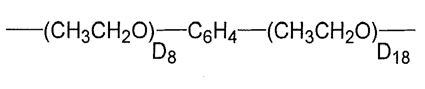
v-b) 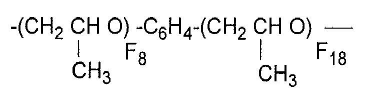
v-c) 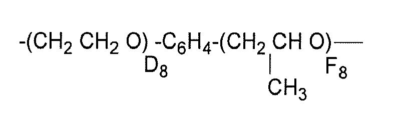
where D₈ and F₈ are as defined above, D₁₈ and F₁₈ are integers each independently comprised between 1 and 40;
vi) a group having the formula -(CH₂CH₂O) _{D8}- where D₈ is as defined above;
vii) a group having the formula 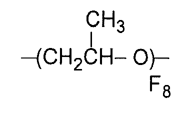 where F₈ is as defined above;
viii) an ethylene oxide and propylene oxide block copolymer having the formula 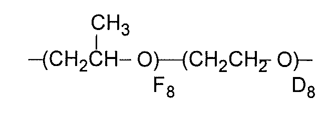 where F₈ and D₈ are as defined above;
ix) an ethylene oxide-propylene oxide-ethylene oxide block copolymer having the formula 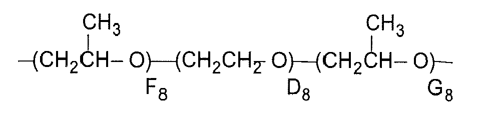 where F₈ and D₈ are as defined above and G₈ is an integer comprised between 1 and 40; wherein:
- if T is carbon, then b is 1,
- if T is nitrogen, then b is 0.

2. The compound according to claim 1, wherein one or more of the following conditions is met:
- n is an integer comprised between 6 and 14, and preferably is equal to 6 or 8;
- R is methyl;
- p is an integer comprised between 2 and 10;
- P is a group selected among:
ii-a) -COOQ
ii-b) -SO₃Q
ii-c) -OSO₃Q
- if Q is ii-f), then the ammonium ion is optionally mono-, di- or trisubstituted with one or more of
ii-f-1) C₁-C₅ alkyl, and
ii-f-2) a group having the formula 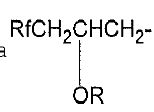 where R and Rf are as defined above,
- p9 is comprised between 2 and 10,
- R₅ is methyl;
- p10 is comprised between 2 and 10;
- p8 is comprised between 2 and 10;

3. The fluorinated compound having the formula (I) according to claim 1, wherein A is zero,
or
wherein A is zero, t=0, v=0, z=0, u=1, R₈=-Cp₈H₂p₈, R₇=CH₃-.

4. The fluorinated compound having the formula (I) according to claim 1, wherein A is 1, or
wherein A is 1 and Y is hydrogen.

5. The fluorinated compound having the formula (I) according to claim 1 selected from the group that consists of:
1a) 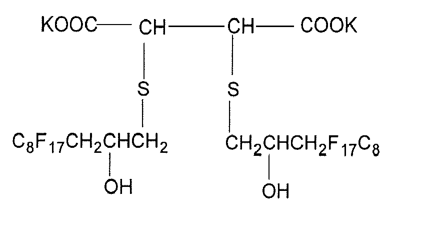
1b) 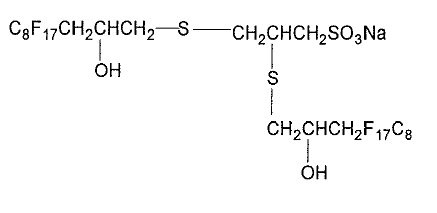
2a) 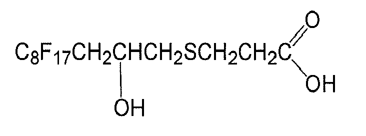
2b) 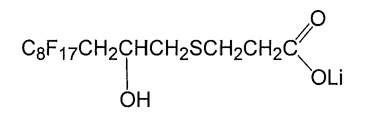
2c) 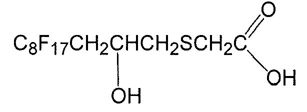
2d) 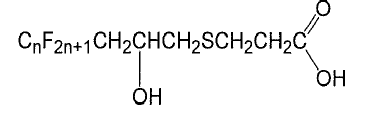
2e) 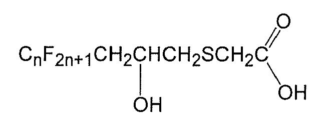
2f) 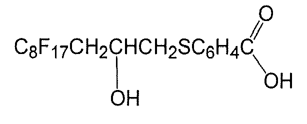
2g) 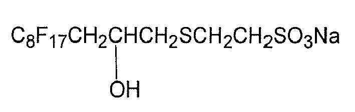
2h) 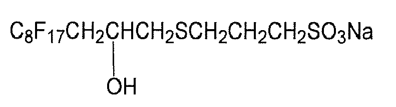
2i) 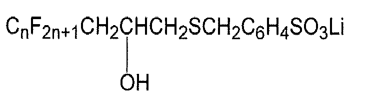
2l) 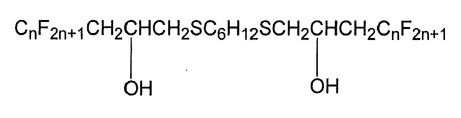
2m) 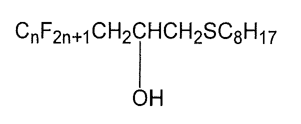 where when n is present, it can assume the values 4, 6, 8, 10, 12, 14, 16, 18.

6. A composition comprising at least one fluorinated compound having the formula (I) according to one or more of claims 1 to 5 in a mixture with a substance selected among:
- one or more solvents selected among water, alcohols, ketones, glycols, esters, dioxane, n-methylpyrrolidinone, tetrahydrofuran, dimethylformamide, toluene, chloroform, methyl chloride, dichloromethane, pure or in turn in a mixture,
- one or more fluorinated and non-fluorinated, amphoteric, anionic and cationic surfactants,
- at least one among paraffins, mixtures of paraffins, mixtures of paraffins and mineral oils, fluorinated compounds, PTFE, boron nitride, molybdenum sulfide, fluorographite, and
- polyethylene powder.

7. A method for synthesizing a compound having the formula (I) as defined in any one of claims 1 to 5, comprising the step of adding a fluorinated epoxide having the general formula (AA) or a mixture of fluorinated epoxides having the formula (AA) 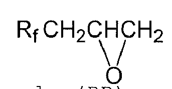 (AA) with variable R_{f} to a compound having the general formula (BB): 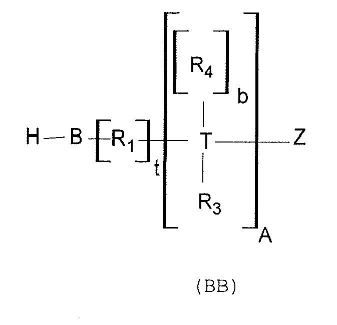 where the variables Rf, B, R₁, T, R₃, R₄, A, t and b are defined as in any one of claims 1 to 5, and
Z is hydrogen or a group having the formula (CC): 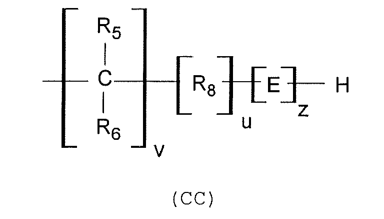 where the variables are defined as in any one of claims 1 to 5,
wherein
when B, and E if present, are both -S-, the reaction temperature is comprised between 20°C and 120°C.

8. The method according to claim 7, wherein if B, and E if present, are both -S- and if the compound having the formula (BB) contains acid groups, the method comprises an additional step for salifying said acid groups by adding inorganic bases such as NaOH, KOH, LiOH, NH₄OH and mixtures thereof or organic bases such as primary, secondary or tertiary amines and mixtures thereof, said salification occurring preferably before addition with the epoxide.

9. The method according to claim 8, wherein the bases are inorganic bases or tertiary amines and said salification step occurs before the reaction of the reagent having the formula (BB) with the fluorinated epoxide (AA).

10. The method according to one or more of claims 7 to 9, further comprising the step of functionalizing the hydroxyl group that derives from the opening of the epoxide to obtain the molecules having the formula (I) in which R is not hydrogen, said step occurring after the reaction of addition.

11. The method according to one or more of claims 7 to 9, further comprising the step of using the hydroxyl group that derives from the opening of the epoxide as a reactive group for the synthesis of polymers, said step occurring after the reaction of addition and being alternative to the group functionalization reaction.

12. The use of a compound having the formula (I) according to one or more of claims 1 to 5, or of a composition according to claim 6, to lower the surface tension of fluid or solid materials to which said compounds or compositions are applied.

13. The use of a compound having the formula (I) according to one or more of claims 1 to 5, or of a composition according to claim 6,
- as nonspecific fluorinated surfactant,
- as solid lubricant to increase the slipperiness and oil-hydro-repellence characteristics of surfaces that constitute hulls of watercraft and the like and/or their cladding materials, sleighs, chutes, ski bases, and the like;
- as additive in polymerization processes in emulsion,
- as corrosion inhibitor,
- as additive for coatings,
- as agent in processes for treating paper and fabrics,
- as additive in enamels and in waxes for floors, for furniture and for shoes,
- as additive in cleaning products,
- as wetting agent,
- as additive in glass antifogging formulas,
- as additive for fire-resistant compositions
- as additive for bases of skis and snow gear.

14. The use according to claim 13, wherein, if it is in a composition, the compound having the formula (I) is used as additive for coatings in a quantity comprised between 0.001% and 10% by weight, or as additive for fire-resistant compositions in a quantity comprised between 0.001% and 10% by weight.

15. The use according to claim 13, wherein the compound having the formula (I) is used as solid lubricant for snow sports gear, in a quantity comprised between 0.3% and 30% by weight if it is in a composition, or as an additive for bases of skis and snow sports gear in combination with polyethylene powder in a quantity comprised between 2% and 10% by weight on the finished composition.
